(19) Europäisches Patentamt / European Patent Office / Office européen des brevets

(11) **EP 3 104 160 B1**

(12) **EUROPEAN PATENT SPECIFICATION**

(45) Date of publication and mention
of the grant of the patent:
**24.11.2021   Bulletin 2021/47**

(21) Application number: **14882044.2**

(22) Date of filing: **07.02.2014**

(51) Int Cl.:
*G01N 13/00* (2006.01)   *G01N 33/487* (2006.01)
*G01N 13/02* (2006.01)

(86) International application number:
**PCT/KR2014/001077**

(87) International publication number:
**WO 2015/119314 (13.08.2015 Gazette 2015/32)**

(54) **METHOD FOR MEASURING FAT CONTENT IN LIQUID BY USING CONTACT AREA DIFFUSION FACTOR**

VERFAHREN ZUR MESSUNG DES FETTGEHALTS IN FLÜSSIGKEIT MITTELS KONTAKTFLÄCHENDIFFUSIONSFAKTOR

PROCÉDÉ DE MESURE DE LA TENEUR EN MATIÈRE GRASSE DANS UN LIQUIDE À L'AIDE DU FACTEUR DE DIFFUSION DE ZONE DE CONTACT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.12.2016   Bulletin 2016/50**

(73) Proprietor: **Femtobiomed Inc.**
**Seongnam-si, Gyeonggi-do 13487 (KR)**

(72) Inventor: **LEE, Sanghyan**
**Pohang-si, Gyeongsangbuk do 791-27 (KR)**

(74) Representative: **Bassil, Nicholas Charles**
**Kilburn & Strode LLP**
**Lacon London**
**84 Theobalds Road**
**London WC1X 8NL (GB)**

(56) References cited:
WO-A1-2013/176757    JP-A- H11 304 685
KR-A- 950 001 300    KR-A- 19980 084 281
KR-A- 20090 097 102    KR-B1- 101 361 072
US-A1- 2008 038 762    US-A1- 2009 299 172

• K Itaya ET AL: "COLORIMETRIC DETERMINATION OF FREE FATTY ACIDS IN BIOLOGICAL FLUIDS", Journal of lipid research, 1 January 1965 (1965-01-01), page 16, XP055398965, UNITED STATES Retrieved from the Internet: URL:http://www.jlr.org/content/6/1/16.full .pdf

• TALLIS G A ET AL: "Lipoprotein profiling by high performance gel chromatography", CLINICA CHIMICA ACTA, ELSEVIER BV, AMSTERDAM, NL, vol. 228, no. 2, 1 August 1994 (1994-08-01), pages 171-179, XP025476476, ISSN: 0009-8981, DOI: 10.1016/0009-8981(94)90287-9 [retrieved on 1994-08-01]

• CHUANJUN LIU ET AL: "Evaporation of sessile water/ethanol drops in a controlled environment", PHYSICAL CHEMISTRY CHEMICAL PHYSICS., vol. 10, no. 47, 1 January 2008 (2008-01-01), page 7150, XP055398823, GB ISSN: 1463-9076, DOI: 10.1039/b808258h

EP 3 104 160 B1

**Description**

Field of the Invention

The present disclosure relates to a method for measuring the fat contents of liquid from the CADF value of the liquid droplet.

Backgroud of the Invention

Liposuction is a plastic operation (surgery) which is the most frequently performed among 21 categories of plastic surgery, over the world including Korea. According to the 2009 report of the ISAPS, over 1.6 millions of liposuctions were performed worldwide.

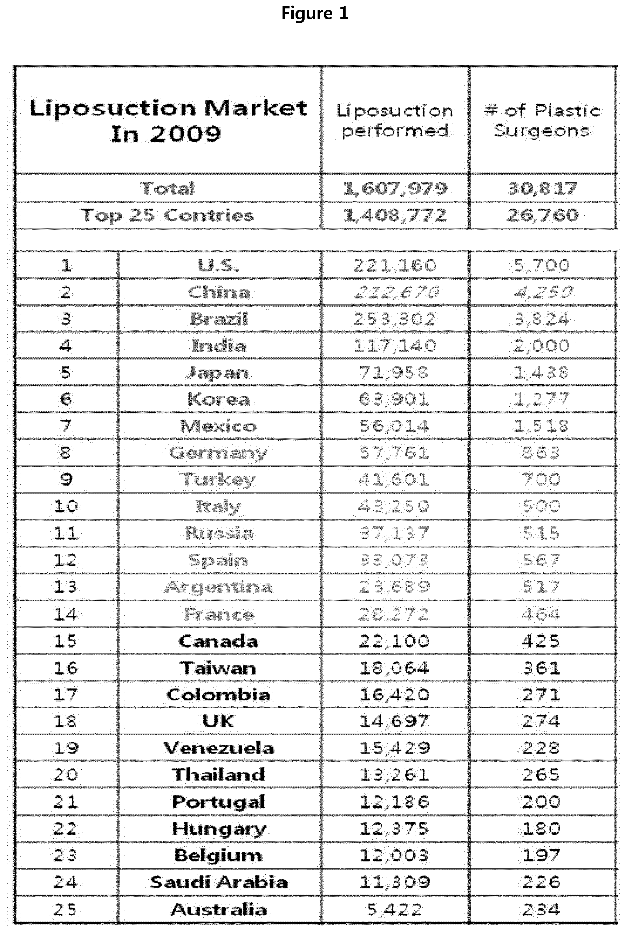

According the above-mentioned report, sixty five thousand liposuctions were performed in Korea (Fig. 1). It is expected that much more liposuctions were performed, considering liposuctions which were not included in the report.

Although liposuction is the most frequently-performed operation among plastic operations, risks that occur during liposuction are not well-known to the public. This is because, on the one hand, mortality accidents due to side effects of liposuction is much fewer than the number of liposuction operations and, on the other hand, most of the mortality accidents are not reported by the press. This is also because FES (fat embolism syndrom) which would be occurred by introduction of fat into blood vessels during liposuction, is known not as a risk of liposuction, but as a medical accident.

Although all the mechamisms of FES has not yet revealed, it is well-known that the most important cause of FES is that fats passing into damaged blood vessels during liposuction may seriously deteriorate the function of the lung or penetrate into other organs, thereby causing death. Presently, it is known that the possibility of inducing FES is proportional to the amount of fats passed into blood stream during liposuction and, accordingly, the mortality risk increases.

Therefore, in order to prevent FES during liposuction, it is necessary to measure the amount of fats passed into blood vessels before, during and after an operation. However, there are no diagnostic apparatuses relating to measuring the amount of fats passed into blood vessels.

Itaya and Ui ("Colorimetric determination of free fatty acids in biological fluids." Journal of Lipid Research 6.1 (1965): 16-20) discloses conditions for chloroform extraction of FFA from blood or other biological fluids.

WO2013176757 (A1) discloses an apparatus and a process for analysis of fatty acids in a plurality of samples by gas chromatography flame ionization detection.

Tallis et al. ("Lipoprotein profiling by high performance gel chromatography." Clinica chimica acta 228.2 (1994): 171-179) discloses high performance gel chromatography (HPGC) to separate lipoproteins on the basis of their size and to generate lipoprotein profiles for plasma collected from patients with different lipoprotein phenotypes. These profiles provide a measurement of low density lipoprotein (LDL)-cholesterol.

US2008038762 (A1) discloses a method and apparatus for analyzing lipoprotein components in blood, where the method includes the step of mixing a serum sample with a fluorescent dye and a self generating gradient material, developing an analyzable mixture in a centrifuge tube under increased gravity via application of external centrifugal force and analyzing the resulting developed mixture to generate a lipoprotein particle component analysis.

Liu et al. ("Evaporation of sessile water/ethanol drops in a controlled environment." Physical Chemistry Chemical Physics 10.47 (2008): 7150-7157) concerns the evaporation rate of two pure, fully miscible liquids (water and ethanol) and their binary mixtures in different ratios at a constant temperature and in controlled vapor pressure of water (relative humidity, RH) and ethanol.

The present inventor has completed the present invention by confirming that the fat content of liquid may be measured from the CADF (contact area diffusion factor) values of a liquid droplet.

Summary of the Invention

The primary object of the present invention is to provide a method for identifying a free fatty acid or LDL in a liquid, according to claim 1, comprising: placing liquid on a water-repellant surface as a droplet (s100); obtaining a magnified image from an image of said droplet (s200); obtaining a contact diameter ($d_0$) from said magnified image (s300); obtaining a contact diameter, $d_{(t)}$, after the lapse of predetermined time ($t$) from said magnified image (s400); and calculating a contact area diffusion factor (CADF) by substituting said $d_{(t)}$ and $d_0$ for the following equation 1 (s500);

$$CADF = \frac{d_{(t)}^2 - d_0^2}{d_0^2} \times 100 \qquad \text{(Equation 1)}$$

where $d_{(t)}$ is the contact diameter of said droplet after the lapse of time, t, and $d_0$ is the initial contact diameter, and said free fatty acid or LDL is determined to be present in said liquid when the value of CADF is positive.

Another object of the present invention is to provide a method for identifying a free fatty acid or LDL in a liquid, according to claim 5, comprising: placing liquid on a water-repellant surface as a droplet (s1100); obtaining a magnified image from an image of said droplet (s1200); obtaining a contact area ($A(0)$) between said repellant surface and said droplet from said magnified image (s1300); and obtaining a contact area, A(t), between said repellant surface and said droplet after the lapse of predetermined time ($t$) from said magnified image (s1400), wherein said free fatty acid or LDL is determined to be present in said liquid when the value of $A(t) - A(0)$ is positive.

Yet another object of the present invention is to provide a method for identifying a free fatty acid or LDL in a liquid based, according to claim 8, comprising: placing liquid on a water-repellant surface as a droplet (s2100); obtaining a magnified image from an image of said droplet (s2200); obtaining a contact diameter (do) from said magnified image (s2300); and obtaining a contact diameter, $d_{(t)}$, after the lapse of predetermined time (t) from said magnified image (s2400), wherein said free fatty acid or LDL is determined to be present in said liquid when the value of $d_{(t)} - do$ is positive.

In other words, the object of the present invention is to provide a method for identifying a free fatty acid or LDL in a liquid by photographing a liquid droplet and calculating the change of the contact area of the liquid droplet and the contact area diffusion factor.

In addition, the present invention is to provide a method for determining the presence of free fatty acid or LDL by using a simple filming equipment where it is required to detect fats such as liposuctions, various orthopedic operations, obesity managements, etc.

Detailed Description of the Invention

The primary object of the present disclosure can be accomplished by providing a method for measuring a free fatty acid or LDL in a liquid based on a contact area diffusion factor, comprising:

placing liquid on a water-repellant surface as a droplet (s100);
obtaining a magnified image from an image of said droplet (s200);
obtaining a contact diameter (do) from said magnified image (s300);
obtaining a contact diameter, $d_{(t)}$, after the lapse of predetermined time ($t$) from said magnified image (s400); and
calculating a contact area diffusion factor (CADF) by substituting said $d_{(t)}$ and $d_0$ for the following equation 1 (s500);

$$\text{CADF} = \frac{d_{(t)}^2 - d_0^2}{d_0^2} \times 100 \qquad \text{(Equation 1)}$$

where $d_{(t)}$ is the contact diameter of said droplet after the lapse of time, t, and $d_0$ is the initial contact diameter; and said free fatty acid or LDL is determined to be present in said liquid when the value of CADF is positive.

As used herein, term "fat" shall include both solid fats (m.p. above 20°C) and liquid fats (i.e., oils) unless otherwise specifically indicated. Fats and oils are generally recognized to be fatty acid triglycerides which are either naturally occurring in vegetable and animal fats and oils, but also include rearranged or randomized fats and oils and interesterified fats and oils.

The term "fatty acid", as used herein, refers to saturatedand or unsaturated (including mono-, di- and poly-unsaturated) straight chain carboxylic acids having from 12 to 24 carbon atoms.

As used herein, the term "trans fat" refers to a type of unsaturated fat containing trans fatty acids. Trans fats increase the LDL cholesterol, while also lowering the HDL cholesterol in blood. The term "trans fatty acid", as used herein, refers to a fatty acid that is commonly produced by the partial hydrogenation of the unsaturated fatty acid vegetable oils. The term "trans" refers to the opposed positioning of hydrogen atoms when unsaturated fats are partially hydrogenated.

As used herein, the term "contact angle" refers to an angle that formed between the surface of a solid and the line tangent to the droplet radius from the point of contact with the solid.

As used herein, the term "body fluid" refers to human or animal serum, plasma, sweat, urine and the like.

The liquid may be a body fluid and the body fluid may be selected from serum, plasma, sweat or urine.

Another object of the present disclosure can be accomplished by providing a method for measuring a free fatty acid or LDL in a liquid based on a contact area diffusion factor, comprising:

placing liquid on a water-repellant surface as a droplet (s1100);
obtaining a magnified image from an image of said droplet (s1200);

obtaining a contact area ($A(0)$) between said repellant surface and said droplet from said magnified image (s1300); and

obtaining a contact area, A(t), between said repellant surface and said droplet after the lapse of predetermined time (t) from said magnified image (s1400),

wherein said free fatty acid or LDL is determined to be present in said body fluid according to whether the value of $A(t) - A(0)$ is positive or negative.

The liquid may be a body fluid and the body fluid may be selected from serum, plasma, sweat or urine.

Yet another object of the present disclosure can be accomplished by providing a method for measuring a free fatty acid or LDL in a liquid based on a contact area diffusion factor, comprising:

placing liquid on a water-repellant surface as a droplet (s2100);
obtaining a magnified image from an image of said droplet (s2200);
obtaining a contact diameter ($d_0$) from said magnified image (s2300); and
obtaining a contact diameter, $d_{(t)}$, after the lapse of predetermined time (t) from said magnified image (s2400),
wherein said free fatty acid or LDL is determined to be present in said body fluid according to whether the value of $d_{(t)}$ - do is positive or negative.

The liquid may be a body fluid and the body fluid may be selected from serum, plasma, sweat or urine.

Advantageous Effects

The present invention provides the following technical effects.

It is possible to determine whether free fatty acids or LDL are present and how much the free fatty acid or LDL exist in a body fluid by calculating the change of the contact area and the contact area diffusion factor (CADF) from an image of a body fluid droplet.

In addition, fat embolism syndrome may be prevented since the presence of fats can be discovered early through the present invention.

Accordingly, free fatty acid or LDL contents may be measured during liposuction, and fat embolism syndrom may be prevented through the present invention. Moreover, the present invention may be used to detect fats when various orthopedic operations and chemical analyses are performed. Further, the present invention may be utilized to monitor an individual's fat metabolism to manage obesity, diet, health care, etc.

Brief Description of the Drawings

Fig. 1 shows statistical data of liposuctions performed worldwide in 2009 by the ISAPA.
Fig. 2 illustrates three surface tensions acting along the contact line between a droplet and a surface of a bottom.
Fig. 3 is a graph that shows the CADF values of the urine containing fats after liposuction and the urine containing no fats before liposuction.
Fig. 4 is a graph that shows the CADF values of the urine samples collected each 0.5 days after liposuction.
Fig. 5 is a graph that shows the CADF values of the urine samples collected each 0.5 days after liposuction.
Fig. 6 is a graph showing the change of the CADF with the change of the concentrations of the urine samples collected after liposuction.
Fig. 7 shows a table that lists the types and concentrations of the free fatty acids, which were measured by gas chromatography (GC), and the free fatty acid were detected by using the CADF values.
Fig. 8 shows the total fatty acids concentration ($C_{CADF}$) and the total fatty acid concentration obtained by adding all the fatty acid concentrations measured by GC.
Fig. 9 is a graph showing the four important fatty acid concentrations and the total fatty acid concentration calculated from the CADF.
Fig. 10 is a graph showing the change of the CADF with diluting the blood plasma samples.
Fig. 11 is a graph showing the LDL (low density lipoprotein) values obtained from the hyperlipidemia test results for the blood samples and the CADF values measured for the same blood samples.

Best Mode for Carrying Out the Invention

Hereinbelow, the present disclosure will be described in greater detail with reference to the following examples and drawings. However, the examples and drawings are given only for illustration of the present disclosure and not to be limiting the present disclosure.

As described in the above, it is known that, as the amount of fats passed into blood stream is larger, the possibility of onset of FES is higher and, accordingly, the mortality risk is higher.

Therefore, in order to prevent FES during liposuction, it is essential to measure quntitatively the amount of the fats passed into blood stream, before, during and after liposuction and, however, there is no diagnostic apparatus in this regard.

The present disclosure provides a method for determining whether a body fluid contains fats from images of the body fluid droplet.

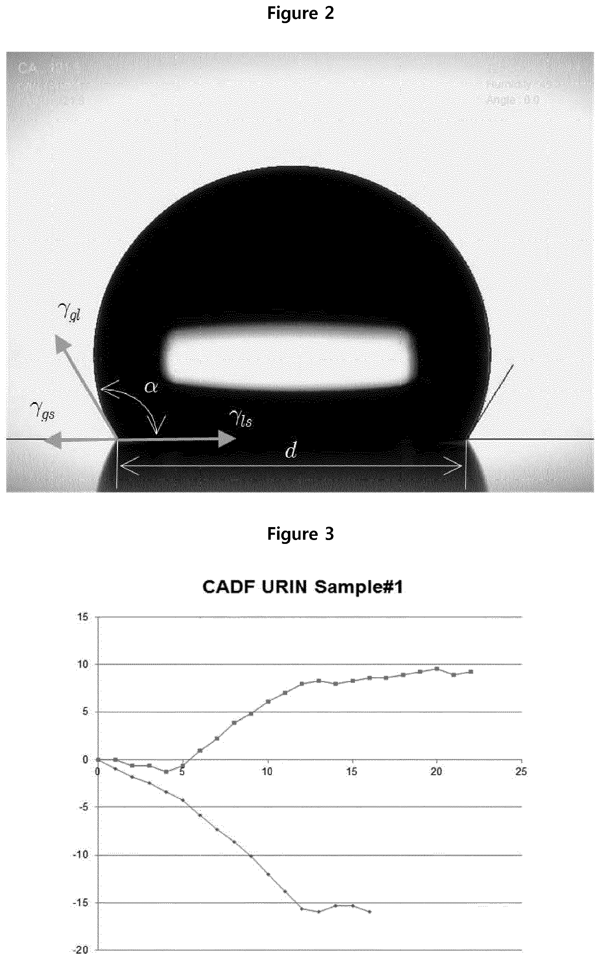

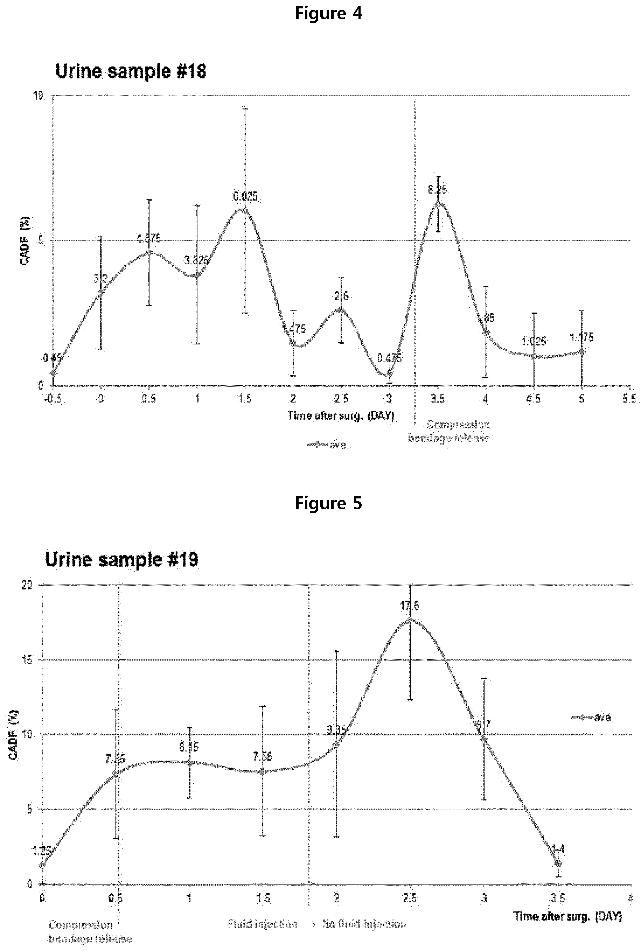

Fig. 2 illustrates three surface tensions acting on the contact line between a droplet and a surface of a bottom, Fig. 3 is a graph that shows the CADF values of the urine containing fats after liposuction and the urine containing no fats before liposuction, Fig. 4 is a graph that shows the CADF values of the urine samples collected each 0.5 days after liposuction, and Fig. 5 is a graph that shows the CADF values of the urine samples collected each 0.5 days after liposuction.

With reference to Fig. 2, blood or urine samples from a patient which were pre-treated were placed, as a micro-droplet, on the specially treated surface and an image of the droplet is shown.

As shown in Fig. 2, when an image of a hydrophilic micro-droplet placed on the solid surface (this droplet is referred to as a sessile droplet) is magnified, it can be understood that a certain contact angle between the micro-droplet and the surface is maintained by surface tensions.

The micro-droplet may be body fluids such as blood, urine, etc. and any other fluid substance.

In Fig. 2, $\gamma_{gl}$ is a surface tension between gas and liquid, $\gamma_{ls}$ is a surface tension between liquid and solid surface, and $\gamma_{gs}$ is a surface tension between gas and solid surface. In addition, $\alpha$ is a contact angle of the micro-droplet, and $d$ is a contact diameter between the micro-droplet and the solid surface.

It is a known technique to measure a contact diameter from an image of a liquid droplet and, thus, detailed description thereof is not provided herein.

Water is evaporated from the micro-droplet as time goes by and, simultaneously, the contact area between the micro-droplet and the solid surface changes.

Considering the changes due to evaporation of water from the micro-droplet, the contact diameter and the contact area decrease as the volume of the micro-droplet decreases.

The contact area decreases during evaporation since the force that tends to maintain the shape of the micro-droplet acts.

That is, the equilibrium between the three surface tensions ($\gamma_{gl}$: a surface tension between gas and liquid, $\gamma_{ls}$: a surface tension between liquid and solid surface, and $\gamma_{gs}$: a surface tension between gas and solid surface) shown in Fig. 2 exists. However, if the contact area does not decrease when the volume of the micro-droplet decreases due to evaporation of water, the equilibrium between the three surface tensions will be broken and force will act in the direction that reduces the contact area and, accordingly, the contact area and the contact diameter (d) will decrease.

If the micro-droplet does not contain free fatty acids or LDL the contact area does not expand (and the contact angle tends to be constant) after evaporation of water. However, if the micro-droplet contains fats, the concentration changes due to evaporation and the surface tension between the liquid and the solid surface changes due to free fatty acid or LDL attached to the solid surface after lapse of time and, therefore, the contact area and angle of the micro-droplet reduce to become flat-shaped.

$$\text{CADF} = \frac{d_{(t)}^2 - d_0^2}{d_0^2} \times 100 \qquad\qquad \text{(Equation 1)}$$

In the equation 1, $d_{(t)}$ is a contact diameter after lapse of time t, and $d_0$ is an initial contact diameter.

As used herein, these degree of change of the contact area is referred to as a contact area diffusion factor (CADF).

If a liquid droplet contains fats and is placed on a water-repellent surface, the water-repellent surface is not sufficiently wetted with water while fats such as oils stronly attach to the water-repellent surface. Therefore, fats contained in the liquid droplet attach to the water-repellent surface.

During attachment of the fats to the water-repellent surface, the surface tension between the liquid droplet and the solid surface ($\gamma_{ls}$) decreases and, thus, the contact angle decreases and the contact area increases. This contact area changes larger when the amount of the fats in the liquid droplet is larger.

When the liquid droplet does not contain free fatty acid or LDL, the contact area or the contact diameter (d) becomes smaller than the initial value; and when the liquid droplet contains free fatty acid or LDL, the contact area or the contact diameter (d) becomes larger than the initial value. The CADF represent this tendency.

As shown in Eq. 1, the CADF equals the difference between the square of the initial contact diameter (do) and the contact diameter $\left(d^2_{(t)}\right)$ which is changing during evaporation divided by the squre of the initial contact area, which is dimensionless. If the contact area decreases, the CADF is negative (-), and *vice versa.* The amount of free fatty acid or LDL content of the liquid droplet may be quantified according to the absolute value of the CADF.

Fig. 3 is a graph that shows the CADF values of the urine containing fats after liposuction and the urine containing no fats before liposuction. It is possible to detect quantitatively the amount of the fats contained in the liquid droplet, and it took about 20 minutes to measure the CADF. The time for the measurement may become shorter or longer, depending on the conditions of the evaporation.

In contrast with the CADF of the urine containing no fats (blue line), the CADF of the urin containing fats (red line) is positive and increasing.

In Fig. 3, the horizontal axis represents time (min) and the vertical axis represents the value of the CADF.

With reference to Fig. 3, the CADF value of urine with no fats are always measured to be negative. That is, the contact area and contact angle of the urine droplet with no fats decrease as water evaporates since the contact angle maintains despite evaporation of water.

However, as shown by the red line in Fig. 3, the CADF of the urine containing fats, e.g., a urine sample obtained from a patient with liposuction, is positive.

This indicates that the shape of the liquid droplet at time t is flatter than that of the initial liquid droplet.

Especially, it has been discovered that changes of other factors except the free fatty acid or LDL content, e.g., ion concentration or pH value, do not influence the CADF.

Therefore, the CADF is positive only when free fatty acid or LDL are contained in the liquid droplet. Thus, it can be determined from the CADF whether or not fats pass into blood streams. Moreover, it can be understood that the amount of free fatty acid or LDL in the liquid droplet corresponds to the absolute value of the CADF.

When the absolute value of the CADF of the blood collected from a patient after liposuction is large, it is possible to determine that much fats passed into blood vessels during liposuction. This may be an indication that a patient may become in a serious condition due to onset of fat embolism syndrome.

Specifically, when fats abruptly pass into blood stream, the fats are discharged through urine, sweat, tears, etc. in order to remove the fats from blood stream. Thus, FES may be diagnosed from body fluids other than blood.

Fig. 4 is a graph that shows the CADF values of the urine samples collected each 0.5 days after liposuction. The average and standard deviation were calculated from four simultaneous measurements per a sample.

The horizontal axis in Fig. 4 represents days after liposuction. The CADFs were measured at every 12 hours from 0.5 days (12 hours) before liposuction to 5 days after liposuction.

With reference to Fig. 4, most of the CADF values are positive. In addition, most of the CADF values measured one month after measuring the samples were relatively small. Therefore, it can be understood that the fat content of the urine decreased with lapse of time.

In the case of the patient in Fig. 4, the CADF value after 3.5 days is the largest for the reason that blood circulation of the patient became good and, thus, the amount of fats discharged through urine increased when compression bandages for the abdomen and the femoral, which were used for suppressing blood circulation after operation (surgery), region were removed. This indicates that medical services influence the amount of fat discharge.

It can be understood that the measurement of the CADF is sensitively influenced by various medical services (e.g., compression of the lesion, supply of infusion solution, etc.). In addition, this demonstrates that the measured value of the CADF sensitively reflects the amount of fats contained in a body fluid.

Fig. 5 is a graph that shows the CADF values of the urine samples collected each 0.5 days after liposuction, from other patient who is not the same patient as the patient in Fig. 4.

The average and standard deviation were calculated from four measurements per a sample. The patient had been hospitalized 1.5 days after surgery (liposuction) with infusion of Ringer's solution, and did not infused with Ringer's solution 2 days after surgery.

The CADF was the largest at 2.5 days since the concentration of the fat increased due to cessation of infusing Ringer's solution. As shown in Fig. 4, fats did not remain in the urine 3.5 days after liposuction.

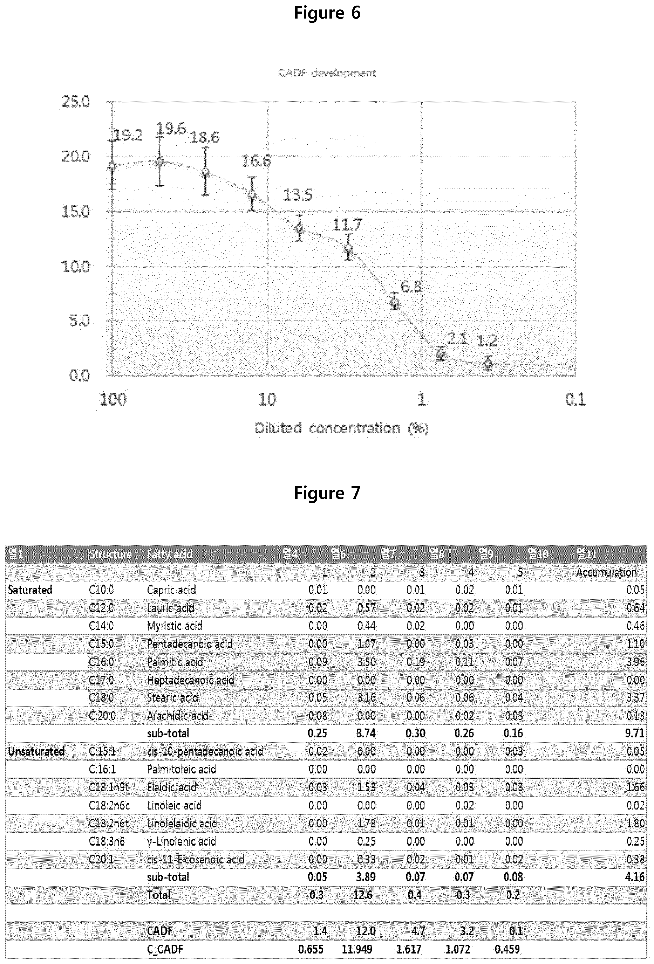

Fig. 6 is a graph showing the change of the CADF with the change of the concentration, diluting with deionized water the urine samples collected after liposuction from 100% to 0.4%. It can be understood from Fig. 6 that fats (e.g., free fatty acids, etc.) that exist in the urine samples may be quantitatively measured from the CADF. From the results of the measurements, the correlation between the CADF and the concentration (C) of the free fatty acids contained in the urine may be obtained as the following equation 2.

$$C = e^{\frac{\text{CADF}-5.53}{3.65}}$$  (Equation 2)

Fig. 7 shows a table that lists the types and concentrations of the free fatty acids, which were measured by gas chromatography (GC), and the free fatty acid were detected by using the CADF values. According to the results of the measurement, the most abundant fatty acids among the fatty acids that newly detected in the urine after liposuction were two saturated fatty acids and two unsaturated fatty acids. Saturated fatty acids and unsaturated trans fatty acids are harmful and are typical materials that cause cardiovascular deseases since these materials are solid at ambient temperature due to their high melting point and, thus, they tend to stick to the vessel wall. Considering the above, any substances that are adhesive may be easily detected from the CADF value.

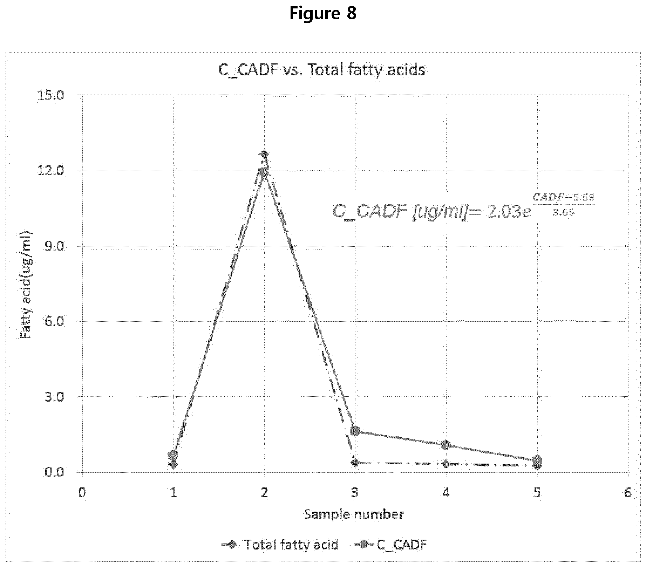

The correlation between the CADF and the amount of fatty acids contained in the urine may be obtained, based on the characteristics of the CADF and the results of analysis of fatty acids contained in the urine, in Fig. 6 and 7. The thus obtained total fatty acids concentration ($C_{CADF}$) and the total fatty acid concentration obtained by adding all the fatty acid concentrations measured by GC are compared in Fig. 8. The total fatty acid concentration in the urine may be calculated from the CADF by the following equation 3.

$$C_{\mathrm{CADF}}\left(ug/ml\right) = 2.03e^{\frac{\mathrm{CADF}-5.53}{3.65}} \qquad \text{(Equation 3)}$$

It can be understood that the total fatty acid concentration obtained from the CADF is well in accord with that obtained from the GC measurement.

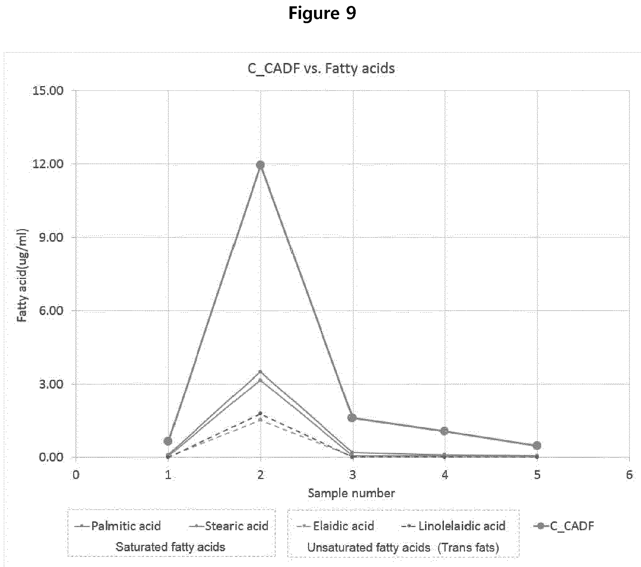

Fig. 9 is a graph showing the four important fatty acid concentrations and the total fatty acid concentration calculated from the CADF. The solid lines (blue and red) in Fig. 9 represent two fatty acids and the dotted lines (blue and grey) represent two unsaturated trans fatty acids.

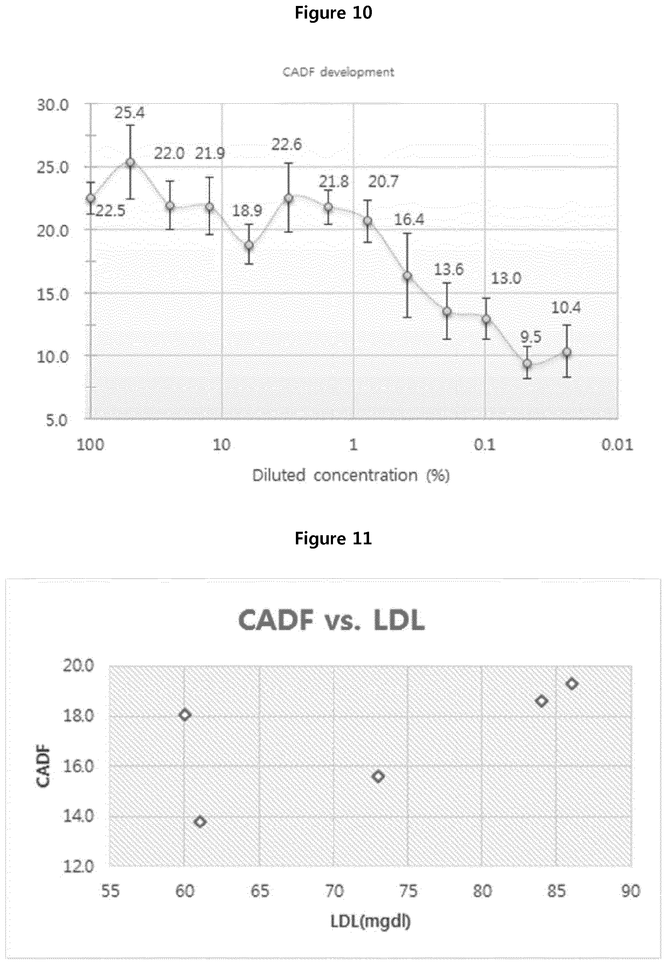

Fig. 10 is a graph showing the change of the CADF with diluting the blood plasma samples. As in the case of the urine, the CADF is proportional to the logarithm of the concentration.

Fig. 11 is a graph showing the LDL (low density lipoprotein) values obtained from the hyperlipidemia test results for the blood samples and the CADF values measured for the same blood samples. It can be understood from Fig. 11 that the LDL value is proportional to the CADF value. In addition, it revealed that the TG (triglycerides) or HDL (high density lipoprotein) value does not have any correlation with the CADF value. Saturated fatty acids or trans fatty acids raise levels of the LDL. Since the CADF is very sensitive to these harmful fatty acids, it can be understood that the CADF value is directly corresponding to the LDL value. Therefore, it is possible to measure the LDL value through measuring the CADF of blood. In addition, the CADF may be used as an indicator for preventing or managing cardiovascular diseases.

Based on the above, it can be understood by a person skilled in the art that the technical effects of the present disclosure are as follows:

Firstly, the present disclosure makes it possible to diagnose and prevent FES by monitoring the fat contents of blood and urine before, during, and after liposuction.

Thus, measures may be taken to a patient before onset of FES.

Secondly, the present disclosure makes it possible to diagnose and prevent FES by monitoring the fat contents of blood and urine before, during, and after orthopedic surgery (operation). Thus, measures may be taken to a patient before onset of FES.

Thirdly, the method for measuring the CADF of the present disclosure may be applied to analytical techniques, for example, measuring the amount of fats contained in a sample. It is very difficult to quantitatively analyze fats through analytical techniques based on capillary columns since it is very hard to attach fluorescent substances to fats and fats do not absorb UV rays. In order to complement the conventional analytical techniques, the method for measuring the CADF of the present disclosure may be combined with the conventional analytical techniques. For example, the CADF according to the present disclosure can be applied to analyses of fats which cannot be measured by UV or fluorescence spectrometry.

Fourthly, the CADF according to the present disclosure can be applied to customized management of body fat in the field of health care/diet/obesity care, through monitoring of the CADF of body fluids such as blood, urine, etc.

Whilst some particular embodiments have been illustrated and described, it will be appreciated by persons skilled in the art that the present invention is not limited by what has been particularly shown by the exemplary embodiments described hereinabove. The invention is defined by the appended claims.

**Claims**

1.   A method for identifying a free fatty acid or LDL in a liquid, comprising:

   placing said liquid on a water-repellant surface as a droplet (s100);
   obtaining a magnified image from an image of said droplet (s200);

obtaining a contact diameter (do) from said magnified image (s300);
obtaining a contact diameter, $d_{(t)}$, after the lapse of predetermined time ($t$) from said magnified image (s400); and
calculating a contact area diffusion factor (CADF) by substituting said $d_{(t)}$ and do for the following equation 1 (s500);

$$\text{CADF} = \frac{d_{(t)}^2 - d_0^2}{d_0^2} \times 100 \qquad \text{(Equation 1)}$$

where $d_{(t)}$ is the contact diameter of said droplet after the lapse of time, t, and $d_0$ is the initial contact diameter, and;
said free fatty acid or LDL is determined to be present in said liquid when the value of CADF is positive.

2. The method of Claim 1, wherein the CADF value is positively correlated to the concentration of free fatty acid or the concentration of LDL.

3. The method of Claim 1, wherein said liquid is a body fluid.

4. The method of Claim 3, wherein said body fluid is selected from the group consisting of serum, plasma, sweat and urine.

5. A method for identifying a free fatty acid or LDL in a liquid, comprising:

placing liquid on a water-repellant surface as a droplet (s1100);
obtaining a magnified image from an image of said droplet (s1200);
obtaining a contact area ($A(0)$) between said repellant surface and said droplet from said magnified image (s1300); and
obtaining a contact area, A(t), between said repellant surface and said droplet after the lapse of predetermined time (t) from said magnified image (s1400),

wherein said free fatty acid or LDL is determined to be present in said liquid when the value of $A(t) - A(0)$ is positive.

6. The method of Claim 5, wherein said liquid is a body fluid.

7. The method of Claim 6, wherein said body fluid is selected from the group consisting of serum, plasma, sweat and urine.

8. A method for identifying a free fatty acid or LDL in a liquid, comprising:

placing liquid on a water-repellant surface as a droplet (s2100);
obtaining a magnified image from an image of said droplet (s2200);
obtaining a contact diameter ($d_0$) from said magnified image (s2300); and
obtaining a contact diameter, $d_{(t)}$, after the lapse of predetermined time ($t$) from said magnified image (s2400),

wherein said free fatty acid or LDL is determined to be present in said liquid when the value of $d_{(t)}$ - do is positive.

9. The method of Claim 8, wherein said liquid is a body fluid.

10. The method of Claim 9, wherein said body fluid is selected from the group consisting of serum, plasma, sweat and urine.

**Patentansprüche**

1. Verfahren zum Identifizieren einer freien Fettsäure oder eines LDL in einer Flüssigkeit, Folgendes umfassend:

Anordnen der Flüssigkeit auf einer wasserabweisenden Oberfläche als ein Tröpfchen (s100);
Ermitteln eines vergrößerten Bilds aus einem Bild des Tröpfchens (s200);
Ermitteln eines Kontaktdurchmessers ($d_0$) aus dem vergrößerten Bild (s300);

Ermitteln eines Kontaktdurchmessers, $d_{(t)}$, nach dem Ablauf einer vorbestimmten Zeit (t) aus dem vergrößerten Bild (s400); und

Berechnen eines Kontaktflächendiffusionsfaktors (CADF) durch Einsetzen von $d_{(t)}$ und $d_0$ in der folgenden Gleichung 1 (s500);

$$CADF = \frac{d_{(t)}^2 - d_0^2}{d_0^2} \times 100 \qquad \text{(Gleichung 1)}$$

wobei $d_{(t)}$ der Kontaktdurchmesser des Tröpfchens nach dem Ablauf der Zeit, t, ist und $d_0$ der anfängliche Kontaktdurchmesser ist und;

die freie Fettsäure oder das LDL als in der Flüssigkeit vorhanden bestimmt wird, wenn der Wert von CADF positiv ist.

2. Verfahren nach Anspruch 1, wobei der CADF-Wert positiv mit der Konzentration der freien Fettsäure oder der Konzentration von LDL korreliert ist.

3. Verfahren nach Anspruch 1, wobei die Flüssigkeit eine Körperflüssigkeit ist.

4. Verfahren nach Anspruch 3, wobei die Körperflüssigkeit aus der Gruppe ausgewählt ist, bestehend aus Serum, Plasma, Schweiß und Urin.

5. Verfahren zum Identifizieren einer freien Fettsäure oder eines LDL in einer Flüssigkeit, Folgendes umfassend:

Anordnen von Flüssigkeit auf einer wasserabweisenden Oberfläche als ein Tröpfchen (s1100);
Ermitteln eines vergrößerten Bilds aus einem Bild des Tröpfchens (s1200);
Ermitteln einer Kontaktfläche (A(0)) zwischen der abweisenden Oberfläche und dem Tröpfchen aus dem vergrößerten Bild (s1300); und
Ermitteln einer Kontaktfläche, A(t), zwischen der abweisenden Oberfläche und dem Tröpfchen nach dem Ablauf einer vorbestimmten Zeit (t) aus dem vergrößerten Bild (s1400),
wobei die freie Fettsäure oder das LDL als in der Flüssigkeit vorhanden bestimmt wird, wenn der Wert von A(t) - A(0) positiv ist.

6. Verfahren nach Anspruch 5, wobei die Flüssigkeit eine Körperflüssigkeit ist.

7. Verfahren nach Anspruch 6, wobei die Körperflüssigkeit aus der Gruppe ausgewählt ist, bestehend aus Serum, Plasma, Schweiß und Urin.

8. Verfahren zum Identifizieren einer freien Fettsäure oder eines LDL in einer Flüssigkeit, Folgendes umfassend:

Anordnen von Flüssigkeit auf einer wasserabweisenden Oberfläche als ein Tröpfchen (s2100);
Ermitteln eines vergrößerten Bilds aus einem Bild des Tröpfchens (s2200);
Ermitteln eines Kontaktdurchmessers ($d_0$) aus dem vergrößerten Bild (s2300); und
Ermitteln eines Kontaktdurchmessers, $d_{(t)}$, nach dem Ablauf der vorbestimmten Zeit (t) aus dem vergrößerten Bild (s2400),
wobei die freie Fettsäure oder das LDL als in der Flüssigkeit vorhanden bestimmt wird, wenn der Wert $d_{(t)} - d_0$ positiv ist.

9. Verfahren nach Anspruch 8, wobei die Flüssigkeit eine Körperflüssigkeit ist.

10. Verfahren nach Anspruch 9, wobei die Körperflüssigkeit aus der Gruppe ausgewählt ist, bestehend aus Serum, Plasma, Schweiß und Urin.

**Revendications**

1. Procédé permettant d'identifier un acide gras libre ou une LDL dans un liquide, comprenant :

le placement dudit liquide sur une surface hydrofuge sous la forme d'une gouttelette (s100) ;

l'obtention d'une image agrandie à partir d'une image de ladite gouttelette (s200) ;

l'obtention d'un diamètre de contact (do) à partir de ladite image agrandie (s300) ;

l'obtention d'un diamètre de contact, $d_{(t)}$, après l'écoulement d'un temps prédéfini (t) à partir de ladite image agrandie (s400) ; et

le calcul d'un facteur de diffusion de zone de contact (CADF) en remplaçant lesdits $d_{(t)}$ et do dans l'équation 1 suivante (s500) ;

$$\text{CADF} = \frac{d_{(t)}^2 - d_0^2}{d_0^2} \times 100$$

(Équation 1)

où $d_{(t)}$ est le diamètre de contact de ladite gouttelette après l'écoulement du temps, t, et $d_0$ est le diamètre de contact initial, et ;

ledit acide gras libre ou ladite LDL sont déterminés comme étant présents dans ledit liquide quand la valeur de CADF est positive.

2. Procédé selon la revendication 1, dans lequel la valeur CADF est positivement corrélée à la concentration d'acide gras libre ou à la concentration de LDL.

3. Procédé selon la revendication 1, dans lequel ledit liquide est un liquide biologique.

4. Procédé selon la revendication 3, dans lequel ledit liquide biologique est choisi dans le groupe constitué par du sérum, du plasma, de la sueur et de l'urine.

5. Procédé permettant d'identifier un acide gras libre ou une LDL dans un liquide, comprenant :

le placement d'un liquide sur une surface hydrofuge sous la forme d'une gouttelette (s1100) ;

l'obtention d'une image agrandie à partir d'une image de ladite gouttelette (s1200) ;

l'obtention d'une zone de contact (A(0)) entre ladite surface répulsive et ladite gouttelette à partir de ladite image agrandie (s1300) ; et

l'obtention d'une zone de contact, A(t), entre ladite surface répulsive et ladite gouttelette après l'écoulement d'un temps prédéfini (t) à partir de ladite image agrandie (s1400),

dans lequel ledit acide gras libre ou ladite LDL sont déterminés comme étant présents dans ledit liquide quand la valeur $A(t) - A(0)$ est positive.

6. Procédé selon la revendication 5, dans lequel ledit liquide est un liquide biologique.

7. Procédé selon la revendication 6, dans lequel ledit liquide biologique est choisi dans le groupe constitué par du sérum, du plasma, de la sueur et de l'urine.

8. Procédé permettant d'identifier un acide gras libre ou une LDL dans un liquide, comprenant :

le placement d'un liquide sur une surface hydrofuge sous la forme d'une gouttelette (s2100) ;

l'obtention d'une image agrandie à partir d'une image de ladite gouttelette (s2200) ;

l'obtention d'un diamètre de contact (do) à partir de ladite image agrandie (s2300) ; et

l'obtention d'un diamètre de contact, $d_{(t)}$, après l'écoulement d'un temps prédéfini (t) à partir de ladite image agrandie (s2400),

dans lequel ledit acide gras libre ou ladite LDL sont déterminés comme étant présents dans ledit liquide quand la valeur de $d_{(t)}$ - do est positive.

9. Procédé selon la revendication 8, dans lequel ledit liquide est un liquide biologique.

10. Procédé selon la revendication 9, dans lequel ledit liquide biologique est choisi dans le groupe constitué par du sérum, du plasma, de la sueur et de l'urine.

**Figure 1**

| Liposuction Market In 2009 | | Liposuction performed | # of Plastic Surgeons |
|---|---|---|---|
| Total | | 1,607,979 | 30,817 |
| Top 25 Contries | | 1,408,772 | 26,760 |
| 1 | U.S. | 221,160 | 5,700 |
| 2 | China | 212,670 | 4,250 |
| 3 | Brazil | 253,302 | 3,824 |
| 4 | India | 117,140 | 2,000 |
| 5 | Japan | 71,958 | 1,438 |
| 6 | Korea | 63,901 | 1,277 |
| 7 | Mexico | 56,014 | 1,518 |
| 8 | Germany | 57,761 | 863 |
| 9 | Turkey | 41,601 | 700 |
| 10 | Italy | 43,250 | 500 |
| 11 | Russia | 37,137 | 515 |
| 12 | Spain | 33,073 | 567 |
| 13 | Argentina | 23,689 | 517 |
| 14 | France | 28,272 | 464 |
| 15 | Canada | 22,100 | 425 |
| 16 | Taiwan | 18,064 | 361 |
| 17 | Colombia | 16,420 | 271 |
| 18 | UK | 14,697 | 274 |
| 19 | Venezuela | 15,429 | 228 |
| 20 | Thailand | 13,261 | 265 |
| 21 | Portugal | 12,186 | 200 |
| 22 | Hungary | 12,375 | 180 |
| 23 | Belgium | 12,003 | 197 |
| 24 | Saudi Arabia | 11,309 | 226 |
| 25 | Australia | 5,422 | 234 |

## Figure 2

## Figure 3

### CADF URIN Sample#1

## Figure 4

Urine sample #18

## Figure 5

Urine sample #19

# Figure 6

# Figure 7

| 열1 | Structure | Fatty acid | 열4 | 열6 | 열7 | 열8 | 열9 | 열10 | 열11 |
|---|---|---|---|---|---|---|---|---|---|
| | | | 1 | 2 | 3 | 4 | 5 | | Accumulation |
| Saturated | C10:0 | Capric acid | 0.01 | 0.00 | 0.01 | 0.02 | 0.01 | | 0.05 |
| | C12:0 | Lauric acid | 0.02 | 0.57 | 0.02 | 0.02 | 0.01 | | 0.64 |
| | C14:0 | Myristic acid | 0.00 | 0.44 | 0.02 | 0.00 | 0.00 | | 0.46 |
| | C15:0 | Pentadecanoic acid | 0.00 | 1.07 | 0.00 | 0.03 | 0.00 | | 1.10 |
| | C16:0 | Palmitic acid | 0.09 | 3.50 | 0.19 | 0.11 | 0.07 | | 3.96 |
| | C17:0 | Heptadecanoic acid | 0.00 | 0.00 | 0.00 | 0.00 | 0.00 | | 0.00 |
| | C18:0 | Stearic acid | 0.05 | 3.16 | 0.06 | 0.06 | 0.04 | | 3.37 |
| | C:20:0 | Arachidic acid | 0.08 | 0.00 | 0.00 | 0.02 | 0.03 | | 0.13 |
| | | sub-total | 0.25 | 8.74 | 0.30 | 0.26 | 0.16 | | 9.71 |
| Unsaturated | C:15:1 | cis-10-pentadecanoic acid | 0.02 | 0.00 | 0.00 | 0.00 | 0.03 | | 0.05 |
| | C:16:1 | Palmitoleic acid | 0.00 | 0.00 | 0.00 | 0.00 | 0.00 | | 0.00 |
| | C18:1n9t | Elaidic acid | 0.03 | 1.53 | 0.04 | 0.03 | 0.03 | | 1.66 |
| | C18:2n6c | Linoleic acid | 0.00 | 0.00 | 0.00 | 0.02 | 0.00 | | 0.02 |
| | C18:2n6t | Linolelaidic acid | 0.00 | 1.78 | 0.01 | 0.01 | 0.00 | | 1.80 |
| | C18:3n6 | γ-Linolenic acid | 0.00 | 0.25 | 0.00 | 0.00 | 0.00 | | 0.25 |
| | C20:1 | cis-11-Eicosenoic acid | 0.00 | 0.33 | 0.02 | 0.01 | 0.02 | | 0.38 |
| | | sub-total | 0.05 | 3.89 | 0.07 | 0.07 | 0.08 | | 4.16 |
| | | Total | 0.3 | 12.6 | 0.4 | 0.3 | 0.2 | | |
| | | | | | | | | | |
| | | CADF | 1.4 | 12.0 | 4.7 | 3.2 | 0.1 | | |
| | | C_CADF | 0.655 | 11.949 | 1.617 | 1.072 | 0.459 | | |

## Figure 8

$$C\_CADF\ [ug/ml] = 2.03e^{\frac{CADF-5.53}{3.65}}$$

# Figure 9

## Figure 10

## Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013176757 A1

- US 2008038762 A1

**Non-patent literature cited in the description**

- **ITAYA ; UI.** Colorimetric determination of free fatty acids in biological fluids. *Journal of Lipid Research,* 1965, vol. 6.1, 16-20
- **TALLIS et al.** Lipoprotein profiling by high performance gel chromatography. *Clinica chimica acta,* 1994, vol. 228.2, 171-179

- **LIU et al.** Evaporation of sessile water/ethanol drops in a controlled environment. *Physical Chemistry Chemical Physics,* 2008, vol. 10.47, 7150-7157